# EUROPEAN PATENT SPECIFICATION

(11) **EP 1 226 170 B1**
(45) Date of publication and mention of the grant of the patent: **30.07.2008**
(21) Application number: 00978238.4
(22) Date of filing: 18.10.2000
(51) Int. Cl.: C07K 14/47, C12N 15/12

(54) **HUMAN CHORDIN AND POLYNUCLEOTIDES ENCODING THE SAME**
MENSCHLICHES CHORDIN UND POLYNUKLEOTIDEN DIE DAFÜR KODIEREN
CHORDIN HUMAINE ET POLYNUCLEOTIDES CODANT POUR CE MEME

(30) Priority: 18.10.1999 US 160106 P; 29.10.1999 US 162547 P
(43) Date of publication of application: 31.07.2002
(73) Proprietor: Lexicon Pharmaceuticals, Inc., The Woodlands, TX 77381 (US)
(72) Inventor: TURNER, C., Alexander, Jr., The Woodlands, TX 77381 (US); DONOHO, Gregory, Portage, MI 49024 (US); NEHLS, Michael, 82131 Stockdorf (DE); HILBUN, Erin, Spring, TX 77379 (US); ZAMBROWICZ, Brian, The Woodlands, TX 77382 (US); SANDS, Arthur, T., The Woodlands, TX 77382 (US)
(74) Representative: Silveston, Judith
(86) International application number: PCT/US2000/028803
(87) International publication number: WO 2001/029084

(56) References cited:
- WO-A-00/09551
- WO-A-00/12708
- WO-A-00/70049
- WO-A-97/39102
- WO-A-98/21332
- WO-A-98/21335
- WO-A-99/15556
- WO-A-99/54353
- JACOBS K ET AL: "A NOVEL METHOD FOR ISOLATING EUKARYOTIC CDNA CLONES ENCODING SECRETED PROTEINS" JOURNAL OF CELLULAR BIOCHEMISTRY - SUPPLEMENT,10 March 1995 (1995-03-10), XP002027246
- NUCLEOTIDE AND PROTEIN SEQUENCS, EBI, UK. 23-06-99. AC AI739159. Homo sapiens cDNA clone IMAGE:2390651 3' similar to SW:CA11_CHICK P02457 PROCOLLAGEN ALPHA 1(I) CHAIN PRECURSOR. ;, mRNA sequence. EST. XP002163462
- NUCLEOTIDE AND PROTEIN SEQUENCS, EBI, UK. 30-08-99. AC = AL110168. Homo sapiens mRNA; cDNA DKFZp586N2124 (from clone DKFZp586N2124); partial cds. XP002163463
- NUCLEOTIDE AND PROTEIN SEQUENCES, EBI, UK 01-11-99 AC = Q9Y3H7. DA141H5.1 (C-TERMINAL PART OF A CHORDIN LIKE PROTEIN WITH VON WILLEBRAND FACTOR TYPE C DOMAINS) (FRAGMENT). Homo sapiens. XP002163464

### SEQUENCE LISTING

<110> Turner, C. Alexander Jr.
   Donoho, Gregory
   Nehls, Michael
   Hilbun, Erin
   Zambrowicz, Brian
   Sands, Arthur T.
<120> Novel Human Proteins and Polynucleotides
   Encoding the Same
<130> LEX-0070-PCT
<150> US 60/160,106
   <151> 1999-10-18
<150> US 60/162,547
   <151> 1999-10-29
<160> 7
<170> FastSEQ for Windows Version 4.0
<210> 1
   <211> 1356
   <212> DNA
   <213> homo sapiens
<400> 1
   atggttcccg aggtgagggt cctctcctcc ttgctgggac tcgcgctgct ctggttcccc      60
   ctggactccc acgctcgagc ccgcccagac atgttctgcc ttttccatgg gaagagatac     120
   tcccccggcg agagctggca cccctacttg gagccacaag gcctgatgta ctgcctgcgc     180
   tgtacctgct cagagggcgc ccatgtgagt tgttaccgcc tccactgtcc gcctgtccac     240
   tgcccccagc ctgtgacgga gccacagcaa tgctgtccca agtgtgtgga acctcacact     300
   ccctctggac tccgggcccc accaaagtcc tgccagcaca acgggaccat gtaccaacac     360
   ggagagatct tcagtgccca tgagctgttc ccctcccgcc tgcccaacca gtgtgtcctc     420
   tgcagctgca cagagggcca gatctactgc ggcctcacaa cctgccccga accaggctgc     480
   ccagcacccc tcccrctgcc agactcctgc tgccargcct gcaaagatga ggcaagtgag     540
   caatcggatg aagaggacag tgtgcagtcg ctccatgggg tgagacatcc tcaggatcca     600
   tgttccagtg atgctgggag aaagagaggc ccgggcaccc cagcccccac tggcctcagc     660
   gcccctctga gcttcatccc tcgccacttc agacccaagg gagcaggcag cacaactgtc     720
   aagatcgtcc tgaaggagaa acataagaaa gcctgtgtgc atggcgggaa gacgtactcc     780
   cacggggagg tgtggcaccc ggccttccgt gccttcggcc ccttgccctg catcctatgc     840
   acctgtgagg atggccgcca ggactgccag cgtgtgacct gtcccaccga gtacccctgc     900
   cgtcaccccg agaaagtggc tgggaagtgc tgcaagattt gcccagagga caaagcagac     960
   cctggccaca gtgagatcag ttctaccagg tgtcccaagg caccgggccg ggtcctcgtc    1020
   cacacatcgg tatccccaag cccagacaac ctgcgtcgct ttgccctgga acacgaggcc    1080
   tcggacttgg tggagatcta cctctggaag ctggtraaag atgaggaaac tgaggctcag    1140
   agaggtgaag tacctggccc aaggccacac agccagaatc ttccacttga ctcagatcaa    1200
   gaaagtcagg aagcaagact tccagaaaga ggcacagcac ttccgactgc tcgctggccc    1260
   ccacgaaggt cactggaacg tcttcctagc ccagaccctg gagctgaagg tcacggccag    1320
   tccagacaaa gtgaccaaga cataacaaag acctaa                              1356
<210> 2
   <211> 451
   <212> PRT
   <213> homo sapiens
<400> 2
   Met Val Pro Glu Val Arg Val Leu Ser Ser Leu Leu Gly Leu Ala Leu
   1               5                   10                  15
   Leu Trp Phe Pro Leu Asp Ser His Ala Arg Ala Arg Pro Asp Met Phe
               20                  25                  30
   Cys Leu Phe His Gly Lys Arg Tyr Ser Pro Gly Glu Ser Trp His Pro
           35                  40                  45
   Tyr Leu Glu Pro Gln Gly Leu Met Tyr Cys Leu Arg Cys Thr Cys Ser
       50                  55                  60
   Glu Gly Ala His Val Ser Cys Tyr Arg Leu His Cys Pro Pro Val His
   65                  70                  75                  80
   Cys Pro Gln Pro Val Thr Glu Pro Gln Gln Cys Cys Pro Lys Cys Val
                   85                  90                  95
   Glu Pro His Thr Pro Ser Gly Leu Arg Ala Pro Pro Lys Ser Cys Gln
               100                 105                 110
   His Asn Gly Thr Met Tyr Gln His Gly Glu Ile Phe Ser Ala His Glu
           115                 120                 125
   Leu Phe Pro Ser Arg Leu Pro Asn Gln Cys Val Leu Cys Ser Cys Thr
       130                 135                 140
   Glu Gly Gln Ile Tyr Cys Gly Leu Thr Thr Cys Pro Glu Pro Gly Cys
   145                 150                 155                 160
   Pro Ala Pro Leu Pro Leu Pro Asp Ser Cys Cys Gln Ala Cys Lys Asp
                   165                 170                 175
   Glu Ala Ser Glu Gln Ser Asp Glu Glu Asp Ser Val Gln Ser Leu His
               180                 185                 190
   Gly Val Arg His Pro Gln Asp Pro Cys Ser Ser Asp Ala Gly Arg Lys
           195                 200                 205
   Arg Gly Pro Gly Thr Pro Ala Pro Thr Gly Leu Ser Ala Pro Leu Ser
       210                 215                 220
   Phe Ile Pro Arg His Phe Arg Pro Lys Gly Ala Gly Ser Thr Thr Val
   225                 230                 235                 240
   Lys Ile Val Leu Lys Glu Lys His Lys Lys Ala Cys Val His Gly Gly
                   245                 250                 255
   Lys Thr Tyr Ser His Gly Glu Val Trp His Pro Ala Phe Arg Ala Phe
               260                 265                 270
   Gly Pro Leu Pro Cys Ile Leu Cys Thr Cys Glu Asp Gly Arg Gln Asp
           275                 280                 285
   Cys Gln Arg Val Thr Cys Pro Thr Glu Tyr Pro Cys Arg His Pro Glu
       290                 295                 300
   Lys Val Ala Gly Lys Cys Cys Lys Ile Cys Pro Glu Asp Lys Ala Asp
   305                 310                 315                 320
   Pro Gly His Ser Glu Ile Ser Ser Thr Arg Cys Pro Lys Ala Pro Gly
                   325                 330                 335
   Arg Val Leu Val His Thr Ser Val Ser Pro Ser Pro Asp Asn Leu Arg
               340                 345                 350
   Arg Phe Ala Leu Glu His Glu Ala Ser Asp Leu Val Glu Ile Tyr Leu
           355                 360                 365
   Trp Lys Leu Val Lys Asp Glu Glu Thr Glu Ala Gln Arg Gly Glu Val
       370                 375                 380
   Pro Gly Pro Arg Pro His Ser Gln Asn Leu Pro Leu Asp Ser Asp Gln
   385                 390                 395                 400
   Glu Ser Gln Glu Ala Arg Leu Pro Glu Arg Gly Thr Ala Leu Pro Thr
                   405                 410                 415
   Ala Arg Trp Pro Pro Arg Arg Ser Leu Glu Arg Leu Pro Ser Pro Asp
               420                 425                 430
   Pro Gly Ala Glu Gly His Gly Gln Ser Arg Gln Ser Asp Gln Asp Ile
           435                 440                 445
   Thr Lys Thr
       450
<210> 3
   <211> 1290
   <212> DNA
   <213> homo sapiens
<400> 3
   atggttcccg aggtgagggt cctctcctcc ttgctgggac tcgcgctgct ctggttcccc      60
   ctggactccc acgctcgagc ccgcccagac atgttctgcc ttttccatgg gaagagatac     120
   tcccccggcg agagctggca cccctacttg gagccacaag gcctgatgta ctgcctgcgc     180
   tgtacctgct cagagggcgc ccatgtgagt tgttaccgcc tccactgtcc gcctgtccac     240
   tgcccccagc ctgtgacgga gccacagcaa tgctgtccca agtgtgtgga acctcacact     300
   ccctctggac tccgggcccc accaaagtcc tgccagcaca acgggaccat gtaccaacac     360
   ggagagatct tcagtgccca tgagctgttc ccctcccgcc tgcccaacca gtgtgtcctc     420
   tgcagctgca cagagggcca gatctactgc ggcctcacaa cctgccccga accaggctgc     480
   ccagcacccc tcccrctgcc agactcctgc tgccargcct gcaaagatga ggcaagtgag     540
   caatcggatg aagaggacag tgtgcagtcg ctccatgggg tgagacatcc tcaggatcca     600
   tgttccagtg atgctgggag aaagagaggc ccgggcaccc cagcccccac tggcctcagc     660
   gcccctctga gcttcatccc tcgccacttc agacccaagg gagcaggcag cacaactgtc     720
   aagatcgtcc tgaaggagaa acataagaaa gcctgtgtgc atggcgggaa gacgtactcc     780
   cacggggagg tgtggcaccc ggccttccgt gccttcggcc ccttgccctg catcctatgc     840
   acctgtgagg atggccgcca ggactgccag cgtgtgacct gtcccaccga gtacccctgc     900
   cgtcaccccg agaaagtggc tgggaagtgc tgcaagattt gcccagagga caaagcagac     960
   cctggccaca gtgagatcag ttctaccagg tgtcccaagg caccgggccg ggtcctcgtc    1020
   cacacatcgg tatccccaag cccagacaac ctgcgtcgct ttgccctgga acacgaggcc    1080
   tcggacttgg tggagatcta cctctggaag ctggtraaag gaatcttcca cttgactcag    1140
   atcaagaaag tcaggaagca agacttccag aaagaggcac agcacttccg actgctcgct    1200
   ggcccccacg aaggtcactg gaacgtcttc ctagcccaga ccctggagct gaaggtcacg    1260
   gccagtccag acaaagtgac caagacataa                                     1290
<210> 4
   <211> 429
   <212> PRT
   <213> homo sapiens
<400> 4
   Met Val Pro Glu Val Arg Val Leu Ser Ser Leu Leu Gly Leu Ala Leu
    1               5                  10                  15
   Leu Trp Phe Pro Leu Asp Ser His Ala Arg Ala Arg Pro Asp Met Phe
               20                  25                  30
   Cys Leu Phe His Gly Lys Arg Tyr Ser Pro Gly Glu Ser Trp His Pro
           35                  40                  45
   Tyr Leu Glu Pro Gln Gly Leu Met Tyr Cys Leu Arg Cys Thr Cys Ser
       50                  55                  60
   Glu Gly Ala His Val Ser Cys Tyr Arg Leu His Cys Pro Pro Val His
   65                  70                  75                  80
   Cys Pro Gln Pro Val Thr Glu Pro Gln Gln Cys Cys Pro Lys Cys Val
                   85                  90                  95
   Glu Pro His Thr Pro Ser Gly Leu Arg Ala Pro Pro Lys Ser Cys Gln
               100                 105                 110
   His Asn Gly Thr Met Tyr Gln His Gly Glu Ile Phe Ser Ala His Glu
           115                 120                 125
   Leu Phe Pro Ser Arg Leu Pro Asn Gln Cys Val Leu Cys Ser Cys Thr
       130                 135                 140
   Glu Gly Gln Ile Tyr Cys Gly Leu Thr Thr Cys Pro Glu Pro Gly Cys
   145                 150                 155                 160
   Pro Ala Pro Leu Pro Leu Pro Asp Ser Cys Cys Gln Ala Cys Lys Asp
                   165                 170                 175
   Glu Ala Ser Glu Gln Ser Asp Glu Glu Asp Ser Val Gln Ser Leu His
               180                 185                 190
   Gly Val Arg His Pro Gln Asp Pro Cys Ser Ser Asp Ala Gly Arg Lys
           195                 200                 205
   Arg Gly Pro Gly Thr Pro Ala Pro Thr Gly Leu Ser Ala Pro Leu Ser
       210                 215                 220
   Phe Ile Pro Arg His Phe Arg Pro Lys Gly Ala Gly Ser Thr Thr Val
   225                 230                 235                 240
   Lys Ile Val Leu Lys Glu Lys His Lys Lys Ala Cys Val His Gly Gly
                   245                 250                 255
   Lys Thr Tyr Ser His Gly Glu Val Trp His Pro Ala Phe Arg Ala Phe
               260                 265                 270
   Gly Pro Leu Pro Cys Ile Leu Cys Thr Cys Glu Asp Gly Arg Gln Asp
           275                 280                 285
   Cys Gln Arg Val Thr Cys Pro Thr Glu Tyr Pro Cys Arg His Pro Glu
       290                 295                 300
   Lys Val Ala Gly Lys Cys Cys Lys Ile Cys Pro Glu Asp Lys Ala Asp
   305                 310                 315                 320
   Pro Gly His Ser Glu Ile Ser Ser Thr Arg Cys Pro Lys Ala Pro Gly
                   325                 330                 335
   Arg Val Leu Val His Thr Ser Val Ser Pro Ser Pro Asp Asn Leu Arg
               340                 345                 350
   Arg Phe Ala Leu Glu His Glu Ala Ser Asp Leu Val Glu Ile Tyr Leu
           355                 360                 365
   Trp Lys Leu Val Lys Gly Ile Phe His Leu Thr Gln Ile Lys Lys Val
       370                 375                 380
   Arg Lys Gln Asp Phe Gln Lys Glu Ala Gln His Phe Arg Leu Leu Ala
   385                 390                 395                 400
   Gly Pro His Glu Gly His Trp Asn Val Phe Leu Ala Gln Thr Leu Glu
                   405                 410                 415
   Leu Lys Val Thr Ala Ser Pro Asp Lys Val Thr Lys Thr
               420                 425
<210> 5
   <211> 1792
   <212> DNA
   <213> homo sapiens
<400> 5
   gccttgcccg gcgctgcgcg ctcggcagac gccggtcctc tccctgcttt ctcctcctct      60
   ccctctcacc ttcccccttt ctttgatcgc ctctcccttc tgctggacct tccttcgtct     120
   ctccatctct ccctcctttc cccgcgttct ctttccacct ttctcttctt cccaccttag     180
   acctcccttc ctgccctcct ttcctgccca ccgctgcttc ctggcccttc tccgaccccg     240
   ctctagcagc agacctcctg gggtctgtgg gttgatctgt ggcccctgtg cctccgtgtc     300
   cttttcgtct cccttcctcc cgactccgct cccggaccag cggcctgacc ctggggaaag     360
   gatggttccc gaggtgaggg tcctctcctc cttgctggga ctcgcgctgc tctggttccc     420
   cctggactcc cacgctcgag cccgcccaga catgttctgc cttttccatg ggaagagata     480
   ctcccccggc gagagctggc acccctactt ggagccacaa ggcctgatgt actgcctgcg     540
   ctgtacctgc tcagagggcg cccatgtgag ttgttaccgc ctccactgtc cgcctgtcca     600
   ctgcccccag cctgtgacgg agccacagca atgctgtccc aagtgtgtgg aacctcacac     660
   tccctctgga ctccgggccc caccaaagtc ctgccagcac aacgggacca tgtaccaaca     720
   cggagagatc ttcagtgccc atgagctgtt cccctcccgc ctgcccaacc agtgtgtcct     780
   ctgcagctgc acagagggcc agatctactg cggcctcaca acctgccccg aaccaggctg     840
   cccagcaccc ctcccgctgc cagactcctg ctgccaagcc tgcaaagatg aggcaagtga     900
   gcaatcggat gaagaggaca gtgtgcagtc gctccatggg gtgagacatc ctcaggatcc     960
   atgttccagt gatgctggga gaaagagagg cccgggcacc ccagccccca ctggcctcag    1020
   cgcccctctg agcttcatcc ctcgccactt cagacccaag ggagcaggca gcacaactgt    1080
   caagatcgtc ctgaaggaga aacataagaa agcctgtgtg catggcggga agacgtactc    1140
   ccacggggag gtgtggcacc cggccttccg tgccttcggc cccttgccct gcatcctatg    1200
   cacctgtgag gatggccgcc aggactgcca gcgtgtgacc tgtcccaccg agtacccctg    1260
   ccgtcacccc gagaaagtgg ctgggaagtg ctgcaagatt tgcccagagg acaaagcaga    1320
   ccctggccac agtgagatca gttctaccag gtgtcccaag gcaccgggcc gggtcctcgt    1380
   ccacacatcg gtatccccaa gcccagacaa cctgcgtcgc tttgccctgg aacacgaggc    1440
   ctcggacttg gtggagatct acctctggaa gctggtaaaa gatgaggaaa ctgaggctca    1500
   gagaggtgaa gtacctggcc caaggccaca cagccagaat cttccacttg actcagatca    1560
   agaaagtcag gaagcaagac ttccagaaag aggcacagca cttccgactg ctcgctggcc    1620
   cccacgaagg tcactggaac gtcttcctag cccagaccct ggagctgaag gtcacggcca    1680
   gtccagacaa agtgaccaag acataacaaa gacctaacag ttgcagatat gagctgtata    1740
   attgttgtta ttatatatta ataaataaga agttgcatta ccctcaaaaa aa              1792
<210> 6
   <211> 918
   <212> DNA
   <213> Homo sapiens
<400> 6
   atgagcctct tcgggcttct cctgctgaca tctgccctgg ccggccagag acaggggact      60
   caggcggaat ccaacctgag tagtaaattc cagttttcca gcaacaagga acagaacgga     120
   gtacaagatc ctcagcatga gagaattatt actgtgtcta ctaatggaag tattcacagc     180
   ccaaggtttc ctcatactta tccaagaaat acggtcttgg tatggagatt agtagcagta     240
   gaggaaaatg tatggataca acttacgttt gatgaaagat ttgggcttga agacccagaa     300
   gatgacatat gcaagtatga ttttgtagaa gttgaggaac ccagtgatgg aactatatta     360
   gggcgctggt gtggttctgg tactgtacca ggaaaacaga tttctaaagg aaatcaaatt     420
   aggataagat ttgtatctga tgaatatttt ccttctgaac cagggttctg catccactac     480
   aacattgtca tgccacaatt cacagaagct gtgagtcctt cagtgctacc cccttcagct     540
   ttgccactgg acctgcttaa taatgctata actgccttta gtaccttgga agaccttatt     600
   cgatatcttg aaccagagag atggcagttg gacttagaag atctatatag gccaacttgg     660
   caacttcttg gcaaggcttt tgtttttgga agaaaatcca gaggagataa ttatgaaaag     720
   gaaaaaaatc tgaagaccaa cttttacaaa tatttggcag agggaaactt ctttaatatt     780
   attatagtta agctattcaa aaagtatcct ttggtacatt atctttcttt cttcttttcc     840
   tttttctctt tatttgcctt ccccccccaa aagtactata caatgtttca agaatgtatg     900
   acatatgact taacttaa                                                   918
<210> 7
   <211> 305
   <212> PRT
   <213> Homo sapiens
<400> 7
   Met Ser Leu Phe Gly Leu Leu Leu Leu Thr Ser Ala Leu Ala Gly Gln
    1               5                  10                  15
   Arg Gln Gly Thr Gln Ala Glu Ser Asn Leu Ser Ser Lys Phe Gln Phe
               20                  25                  30
   Ser Ser Asn Lys Glu Gln Asn Gly Val Gln Asp Pro Gln His Glu Arg
           35                  40                  45
   Ile Ile Thr Val Ser Thr Asn Gly Ser Ile His Ser Pro Arg Phe Pro
       50                  55                  60
   His Thr Tyr Pro Arg Asn Thr Val Leu Val Trp Arg Leu Val Ala Val
   65                  70                  75                  80
   Glu Glu Asn Val Trp Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Thr Ile Leu Gly Arg Trp Cys Gly Ser Gly Thr
           115                 120                 125
   Val Pro Gly Lys Gln Ile Ser Lys Gly Asn Gln Ile Arg Ile Arg Phe
       130                 135                 140
   Val Ser Asp Glu Tyr Phe Pro Ser Glu Pro Gly Phe Cys Ile His Tyr
   145                 150                 155                 160
   Asn Ile Val Met Pro Gln Phe Thr Glu Ala Val Ser Pro Ser Val Leu
                   165                 170                 175
   Pro Pro Ser Ala Leu Pro Leu Asp Leu Leu Asn Asn Ala Ile Thr Ala
               180                 185                 190
   Phe Ser Thr Leu Glu Asp Leu Ile Arg Tyr Leu Glu Pro Glu Arg Trp
           195                 200                 205
   Gln Leu Asp Leu Glu Asp Leu Tyr Arg Pro Thr Trp Gln Leu Leu Gly
       210                 215                 220
   Lys Ala Phe Val Phe Gly Arg Lys Ser Arg Gly Asp Asn Tyr Glu Lys
   225                 230                 235                 240
   Glu Lys Asn Leu Lys Thr Asn Phe Tyr Lys Tyr Leu Ala Glu Gly Asn
                   245                 250                 255
   Phe Phe Asn Ile Ile Ile Val Lys Leu Phe Lys Lys Tyr Pro Leu Val
               260                 265                 270
   His Tyr Leu Ser Phe Phe Phe Ser Phe Phe Ser Leu Phe Ala Phe Pro
           275                 280                 285
   Pro Gln Lys Tyr Tyr Thr Met Phe Gln Glu Cys Met Thr Tyr Asp Leu
       290                 295                 300
   Thr
   305

## Description

### 1. INTRODUCTION

The present application claims the benefit of U.S. Provisional Application Serial Nos: 60/160,106 and 60/162,547 which were filed on October 18, 1999 and October 29, 1999, respectively, and are herein incorporated by reference in their entirety.

The present invention relates to the discovery, identification, and characterization of novel human polynucleotides that encode proteins, and, more particularly, secreted proteins. The invention encompasses the described polynucleotides, host cell expression systems, the encoded proteins, fusion proteins, polypeptides and peptides, antibodies to the encoded proteins and peptides, and genetically engineered animals that lack the disclosed sequences, or over express the disclosed sequences, or antagonists and agonists of the proteins, and other compounds that modulate the expression or activity of the proteins encoded by the disclosed sequences that can be used for diagnosis, drug screening, clinical trial monitoring, the treatment of physiological or behavioral disorders, cancer, and infectious disease.

### 2. BACKGROUND OF THE INVENTION

Proteins are integral components of the various systems used by the body to monitor and regulate different bodily functions. In particular, secreted proteins, or circulating fragments or portions of other proteins, are often involved in regulating and maintaining a wide variety of biological and physiological processes. Often, such processes are mediated by protein ligands that interact with corresponding membrane receptor proteins that activate signal transduction and other pathways that control cell physiology, chemical release and communication, and gene expression. As such, ligand/receptor interactions constitute ideal targets for drug intervention and for the design of therapeutic agents.

### 3. SUMMARY OF THE INVENTION

The present invention relates to the discovery, identification, and characterization of nucleotides that encode novel human proteins, polypeptides, and peptides, and the corresponding amino acid sequences thereof.

The novel human proteins (NHPs) described for the first time herein share structural motifs typical of human secreted proteins. Two of the novel human nucleic acid sequences described herein, encode proteins/open reading frames (ORFs) of 451 and 429 amino acids in length (see SEQ ID NOS: 2 and 4 respectively). These novel human proteins (NHPs) described for the first time herein share structural similarity with animal chordins, NEL protein, and thrombospondin. Chordins are developmentally active proteins that are antagonists of bone morphogenic protein (BMP) 4, and serve as targets for proteolytic cleavage by BMP-1. Chordin has been implicated in developmental regulation during gastrulation and skeletogenesis (Piccolo et al. 1996. Cell, 86: 589-598.). The regions of the described proteins that constitute the chordin-like domains also display marked similarity with human NEL protein and animal thrombospondins. In addition to development, these proteins have been associated with biological activities such as, for example, the inhibition of angiogenesis, clotting, and adrenal secretion.

Described herein are nucleotides presented in the Sequence Listing, host cells expressing such nucleotides, the expression products of such nucleotides, and: (a) nucleotides that encode mammalian homologs of the described sequences, including the specifically described NHPs, and the NHP products; (b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence in deleted; and (d) nucleotides that encode chimeric fusion proteins containing all or a portion of a coding region of an NHP, or one of its domains (e.g., a receptor binding domain, accessory protein/self-association domain, etc.) fused to another peptide or polypeptide.

Further described are agonists and antagonists of the NHPs, including small molecules, large molecules, mutant NHPs, or portions thereof that compete with native NHP, and antibodies, as well as nucleotide sequences that can be used to inhibit the expression of the described NHPs (e.g., antisense and ribozyme molecules, and gene or regulatory sequence replacement constructs) or to enhance the expression of the described NHP sequences (e.g., expression constructs that place the described sequence under the control of a strong promoter system), and transgenic animals that express a NHP transgene. Additionally, "knock-out" animals are contemplated (which can be conditional) that have been engineered such that they do not express a functional NHP gene (see, for example, PCT Applic. No. PCT/US98/03243, filed February 20, 1998, herein incorporated by reference). Another aspect of the present invention includes cells and animals that having specifically engineered mutations (point mutations, over expression of an NHP gene, etc.) the genes encoding the presently described NHPs.

Further described are methods of using the described NHPs and/or NHP products for the identification of compounds that modulate, i.e., act as agonists or antagonists, of NHP expression and/or NHP product activity. Like the described NHPs, such compounds can be used as therapeutic agents for the treatment of any of a wide variety of symptomatic representations of biological disorders or imbalances.

### 4. DESCRIPTION OF THE SEQUENCE LISTING AND FIGURES

The Sequence Listing provides the sequence of four nucleotide sequences and the novel amino acid sequences encoded by three of them. SEQ ID NO:5 describes a NHP ORF and flanking sequences.

### 5. DETAILED DESCRIPTION OF THE INVENTION

The NHPs, described for the first time herein, are novel proteins that are expressed in, *inter alia,* human cell lines, gene trapped human cell lines and cervix cells. When secreted, the NHPs typically exert physiological effect by interacting with receptors to produce a biological effect (such as, for example, signal transduction). Consequently, interfering with the binding of a NHP product to its cognate receptor effects NHP-mediated processes. Alternatively, enhancing the concentration of a NHP product *in vivo,* can boost the effects/activity levels of such NHP affected processes. Yet another alternative is that the described NHPs, or portions thereof, can act as hormones (or peptide hormones), enzymes, or receptor/ligand antagonists and used accordingly.

Because secreted proteins are considered to be more likely to effect some biological activity, the genes encoding such proteins (and the proteins encoded thereby as well as the uses and formulations thereof) have been the subjects of intense scientific/commercial scrutiny (see, for example, Applic. Ser. Nos. PCT/US98/04858 (from 60/068,368, 60/057,765, 60/048,970, 60/040,762 and others listed on the face of the application) filed March 12, 1998, 09/040,963, filed March 18, 1998, PCT/US98/05255 (corresponding to 60/041,263), filed March 18, 1998, all of which are herein incorporated by reference in their entirety). The presently described NHPs have structural motifs consistent with other human secreted proteins.

Described is the use of the described NHP nucleotides, NHPs, and peptides, as well as antibodies, preferably humanized monoclonal antibodies, or binding fragments, domains, or fusion proteins thereof, or antiidotypic variants derived therefrom, that bind NHPs (which can, for example, also act as NHP agonists or antagonists), other antagonists that inhibit binding activity or expression, or agonists that activate NHP receptor activity or increase NHP expression, in the diagnosis and/or treatment of disease.

In particular, what is described in the subsections below encompasses the described NHPs, or peptides corresponding to functional domains of NHPs, mutated NHPs, truncated NHPs, mature cleavage products of NHPs (which may occur, prior to, during, or subsequent to secretion), or deleted NHPs (e.g., NHPs missing one or more functional regions, domains, or portions thereof), NHP fusion proteins (e.g., a NHP or a functional domain of a NHP fused to an unrelated protein or peptide such as an immunoglobulin constant region, *i.e.,* IgFc), nucleotide sequences encoding such products, and host cell expression systems that can produce such NHP products.

Described are also antibodies and anti-idiotypic antibodies (including Fab fragments), antagonists and agonists of the NHP, as well as compounds or nucleotide constructs that inhibit expression of a NHP gene (transcription factor inhibitors, antisense and ribozyme molecules, or gene or regulatory sequence replacement constructs), or promote expression of NHPs *(e.g.,* expression constructs in which NHP coding sequences are operatively associated with expression control elements such as promoters, promoter/enhancers, etc.). The invention also relates to host cells and animals genetically engineered to express the NHPs (or mutant variants thereof) or to inhibit, or "knock-out", expression of an animal homolog of an endogenous NHP gene.

As putative secreted proteins/peptides, signal peptides associated with the described amino acid sequences may be typically cleaved during secretion of the mature protein products. Analysis of the described proteins/peptides reveals the presence of predicted signal cleavage sites between about 13 and about 53 amino acids into the described proteins (from the initiation methionine). Computer predictions of signal peptidase cleavage sites being less than absolutely accurate, an additional aspect of the present invention includes any and all mature cleavage products remaining after removal of between about the first 10 and about the first 55 amino acids, or any number in-between (as applicable given the length of the described protein), that leaves (for secretion) at least about 3, and preferably at least about 6 to 20, or more, amino acids of the protein product originally encoded by the described NHP sequences (for secretion).

The NHPs or peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for an NHP, but can also identify compounds that trigger NHP-mediated signal transduction.

Finally, the NHP products (especially soluble derivatives such as peptides corresponding to the, and fusion protein products (especially NHP-Ig fusion proteins, i.e., fusions of a NHP, or a domain of a NHP, to an IgFc), antibodies and anti-idiotypic antibodies (including Fab fragments), antagonists or agonists (including compounds that modulate signal transduction which may act on downstream targets in a NHP-mediated signal transduction pathway) can be used to directly treat diseases or disorders. For example, the administration of an effective amount of soluble NHP, or a NHP-IgFc fusion protein or an anti-idiotypic antibody (or its Fab) that mimics the NHP could activate or neutralize the endogenous NHP receptor. Nucleotide constructs encoding such NHP products can be used to genetically engineer host cells to express such products in vivo; these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders, boosting immune function, or combating infectious agents.

Various aspects of the invention are described in greater detail in the subsections below.

### 5.1 THE NHP SEQUENCES

The cDNA sequences (SEQ ID NOS: 1, 3 and 6) and the corresponding deduced amino acid sequences (SEQ ID NOS: 2, 4 and 7) of the described NHPs are presented in the Sequence Listing. The two NHP nucleotides described in SEQ ID NOS: 1 and 3 were obtained from human gene trapped sequence tags and polynucleotide isolated from a human adrenal gland library (Edge Biosystems, Gaithersburg, MD). SEQ ID NO:5 shows a polynucleotide sequence encoding a NHP ORF as well as 5' and 3' flanking regions.

The NHPs disclosed herein include: (a) the human DNA sequences presented in the Sequence Listing, and additionally contemplate any nucleotide sequence encoding a contiguous and functional NHP open reading frame (ORF) that hybridizes to a complement of the DNA sequences presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1x55C/0.1% SDS at 68°C (Ausubel F.M. et al., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of the DNA sequences that encode and express an amino acid sequence presented in the Sequence Listing under moderately stringent conditions, *e.g.,* washing in 0.2xSSC/0.1% SDS at 42°C (Ausubel et al., 1989, *supra),* yet which still encode a functionally equivalent NHP product. Functional equivalents of NHPs include naturally occurring NHPs present in other species, and mutant NHPs whether naturally occurring or engineered. The invention also includes degenerate variants of the disclosed sequences.

Disclosed are also nucleic acid molecules, preferably DNA molecules, that hybridize to, and are therefore the complements of, the described NHP nucleotide sequences. Such hybridization conditions may be highly stringent or less highly stringent, as described above. In instances wherein the nucleic acid molecules are deoxyoligonucleotides ("DNA oligos"), such molecules (and particularly about 16 to about 100 base long, about 20 to about 80, or about 34 to about 45 base long, or any variation or combination of sizes represented therein incorporating a contiguous region of sequence first disclosed in the Sequence Listing, can be used in conjunction with the polymerase chain reaction (PCR) to screen libraries, isolate clones, and prepare cloning and sequencing templates, etc.

Alternatively, the oligonucleotides can be used as hybridization probes. For oligonucleotide probes, highly stringent conditions may refer, *e.g.*, to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences, also useful for NHP gene regulation.

Additionally, the antisense oligonucleotides may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

The antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

The antisense oligonucleotide may be an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-0-methylribonucleotide (Inoue et al., 1987, Nucl. Acids Res. 15:6131-6148), or a chimeric RNA-DNA analogue (Inoue et al., 1987, FEBS Lett. 215:327-330).

Oligonucleotides may be synthesized by standard methods known in the art, *e.g.* by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook et al., 1989, Molecular Cloning, A Laboratory Manual (and periodic updates thereof), Cold Springs Harbor Press, N.Y.; and Ausubel et al., 1989, Current Protocols in Molecular Biology, Green Publishing Associates and Wiley Interscience, N.Y.

Alternatively, suitably labeled NHP nucleotide probes may be used to screen a human genomic library using appropriately stringent conditions or by PCR. The identification and characterization of human genomic clones is helpful for identifying polymorphisms, determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g.*, splice acceptor and/or donor sites), etc., that can be used in diagnostics and pharmacogenomics.

Further, a NHP gene homolog may be isolated from nucleic acid of the organism of interest by performing PCR using two degenerate oligonucleotide primer pools designed on the basis of amino acid sequences within the NHP product disclosed herein. The template for the reaction may be total RNA, mRNA, and/or cDNA obtained by reverse transcription of mRNA prepared from, for example, human or non-human cell lines or tissue, such as choroid plexus, known or suspected to express a NHP gene allele.

The PCR product may be subcloned and sequenced to ensure that the amplified sequences represent the sequence of the desired NHP gene. The PCR fragment may then be used to isolate a full length cDNA clone by a variety of methods. For example, the amplified fragment may be labeled and used to screen a cDNA library, such as a bacteriophage cDNA library. Alternatively, the labeled fragment may be used to isolate genomic clones via the screening of a genomic library.

PCR technology may also be utilized to isolate full length cDNA sequences. For example, RNA may be isolated, following standard procedures, from an appropriate cellular or tissue source (*i.e.,* one known, or suspected, to express a NHP gene, such as, for example, brain tissue). A reverse transcription (RT) reaction may be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment may easily be isolated. For a review of cloning strategies which may be used, see e.g., Sambrook et *al.,* 1989, *supra.*

A cDNA of a mutant NHP gene may be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of the normal NHP allele, the mutation(s) responsible for the loss or alteration of function of the mutant NHP gene product can be ascertained.

Alternatively, a genomic library can be constructed using DNA obtained from an individual suspected of or known to carry the mutant NHP allele, or a cDNA library can be constructed using RNA from a tissue known, or suspected, to express the mutant NHP allele. A normal NHP gene, or any suitable fragment thereof, can then be labeled and used as a probe to identify the corresponding mutant NHP allele in such libraries. Clones containing the mutant NHP gene sequences may then be purified and subjected to sequence analysis according to methods well known to those of skill in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue may be expressed and screened using standard antibody screening techniques in conjunction with antibodies raised against the normal NHP product, as described, below, in Section 5.3. (For screening techniques, see, for example, Harlow, E. and Lane, eds., 1988, "Antibodies: A Laboratory Manual", Cold Spring Harbor Press, Cold Spring Harbor.)

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function *(e.g.,* as a result of a missense or a frameshift mutation), a polyclonal set of antibodies to NHP are likely to cross-react with the mutant NHP sequence product. Library clones detected via their reaction with such labeled antibodies can be purified and subjected to sequence analysis according to methods well known to those of skill in the art.

Disclosed are also nucleotide sequences that encode mutant NHPs, peptide fragments of the NHPs, truncated NHPs, and NHP fusion proteins. These include, but are not limited to nucleotide sequences encoding mutant NHPs described in section 5.2 *infra;* polypeptides or peptides corresponding to one or more domains of the NHP or portions of these domains; truncated NHPs in which one or more of the domains is deleted, or a truncated nonfunctional NHP. Nucleotides encoding fusion proteins may include, but are not limited to, full length NHP sequences, truncated NHPs, or nucleotides encoding peptide fragments of a NHP fused to an unrelated protein or peptide, such as for example, a NHP domain fused to an Ig Fc domain which increases the stability and half life of the resulting fusion protein (*e.g.,* NHP-Ig) in the bloodstream; or an enzyme such as a fluorescent protein or a luminescent protein which can be used as a marker.

Disclosed are (a) DNA vectors that contain any of the foregoing NHP coding sequences and/or their complements (i.e., antisense); (b) DNA expression vectors that contain any of the foregoing NHP coding sequences operatively associated with a regulatory element that directs the expression of the coding sequences; (c) genetically engineered host cells that contain any of the foregoing NHP coding sequences operatively associated with a regulatory element that directs the expression of the coding sequences in the host cell; and (d) genetically engineered host cells that express an endogenous NHP sequence under the control of an exogenously introduced regulatory element (*i.e.,* gene activation).

As used herein, regulatory elements include, but are not limited to, inducible and non-inducible promoters, enhancers, operators and other elements known to those skilled in the art that drive and regulate expression. Such regulatory elements include but are not limited to the human cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

### 5.2 NHP PROTEINS AND POLYPEPTIDES

NHPs, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, for the identification of other cellular sequence products related to a NHP, as reagents in assays for screening for compounds that can be as pharmaceutical reagents useful in the therapeutic treatment of mental, biological, or medical disorders and disease.

The Sequence Listing discloses the amino acid sequences encoded by the described NHP sequences. The NHPs have initiator methionines in DNA sequence contexts consistent with translation initiation sites, followed by hydrophobic signal sequences typical of secreted proteins.

The NHP amino acid sequences disclosed herein include the nucleotide and amino acid sequences presented in the Sequence Listing as well as analogues and derivatives thereof. Further, corresponding NHP homologues from other species are disclosed herein. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell et al. eds., Scientific American Books, New York, NY, herein incorporated by reference) are generically representative of all the various permutations and combinations of nucleic acid sequences that can encode such amino acid sequences.

Disclosed are also proteins that are functionally equivalent to the NHPs encoded by the nucleotide sequences described in Section 5.1, as judged by any of a number of criteria, including, but not limited to, the ability to bind a receptor of a NHP , the ability to effect an identical or complementary signal transduction pathway, a change in cellular metabolism (*e.g.*, ion flux, tyrosine phosphorylation, etc.), or change in phenotype when the NHP equivalent is similarly expressed or mutated in an appropriate cell type (such as the amelioration, prevention or delay of a biochemical, biophysical, or overt phenotype). Such functionally equivalent NHP proteins include, but are not limited to, additions or substitutions of amino acid residues within the amino acid sequence encoded by the NHP nucleotide sequences described above in Section 5.1, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

While random mutations can be made to NHP DNA (using random mutagenesis techniques well known to those skilled in the art) and the resulting mutant NHPs tested for activity, site-directed mutations of the NHP coding sequence can be engineered (using site-directed mutagenesis techniques well known to those skilled in the art) to generate mutant NHPs with increased function, *e.g.*, higher receptor binding affinity, decreased function, and/or increased physiological half-life, and increased signal transduction triggering. One starting point for such analysis is by aligning the disclosed human sequences with corresponding gene/protein sequences from, for example, other mammals in order to identify amino acid sequence motifs that are conserved between different species. Non-conservative changes can be engineered at variable positions to alter function, signal transduction capability, or both. Alternatively, where alteration of function is desired, deletion or non-conservative alterations of the conserved regions (i.e., identical amino acids) can be engineered. For example, deletion or non-conservative alterations (substitutions or insertions) of the various conserved transmembrane domains.

Other mutations to the NHP coding sequence can be made to generate NHPs that are better suited for expression, scale up, etc. in the host cells chosen. For example, cysteine residues can be deleted or substituted with another amino acid in order to eliminate disulfide bridges; N-linked glycosylation sites can be altered or eliminated to achieve, for example, expression of a homogeneous product that is more easily recovered and purified from yeast hosts which are known to hyperglycosylate N-linked sites. To this end, a variety of amino acid substitutions at one or both of the first or third amino acid positions of any one or more of the glycosylation recognition sequences which occur in the NHP (N-X-S or N-X-T), and/or an amino acid deletion at the second position of any one or more such recognition sequences in the NHP will prevent glycosylation of the NHP at the modified tripeptide sequence. (See, *e.g.,* Miyajima et al., 1986, EMBO J. 5(6):1193-1197).

Peptides corresponding to one or more fragment or domain of a NHP, truncated or deleted NHPs, as well as fusion proteins in which a full length NHP, a NHP peptide, or truncated NHP is fused to an unrelated protein, are also disclosed and can be designed on the basis of the presently disclosed NHP nucleotide and NHP amino acid sequences. Typically, a peptide can have as few as three amino acids, but preferably at least about 6 amino acids, more preferably at least about 12 amino acids and up to about 80 amino acids. Fusion proteins include, but are not limited to, IgFc fusions which stabilize the NHP protein or peptide and prolong half-life *in vivo;* or fusions to any amino acid sequence that allows the fusion protein to be anchored to the cell membrane; or fusions to an enzyme, fluorescent protein, or luminescent protein which provide a marker function.

While the NHPs and peptides can be chemically synthesized (e.g., see Creighton, 1983, Proteins: Structures and Molecular Principles, W.H. Freeman & Co., N.Y.), large polypeptides derived from a NHP and full length NHPs can be advantageously produced by recombinant DNA technology using techniques well known in the art for expressing nucleic acid containing NHP sequences and/or coding sequences. Such methods can be used to construct expression vectors containing a NHP nucleotide sequences described in Section 5.1 and appropriate transcriptional and translational control signals. These methods include, for example, *in vitro* recombinant DNA techniques, synthetic techniques, and *in vivo* genetic recombination. See, for example, the techniques described in Sambrook et *al.,* 1989, *supra,* and Ausubel et *al.,* 1989, *supra.* Alternatively, RNA corresponding to all or a portion of a transcript encoded by a NHP nucleotide sequence may be chemically synthesized using, for example, synthesizers. See, for example, the techniques described in "Oligonucleotide Synthesis", 1984, Gait, M.J. ed., IRL Press, Oxford, which is incorporated by reference herein in its entirety.

A variety of host-expression vector systems may be utilized to express the NHP nucleotide sequences of the invention. Where the NHP peptide or polypeptide is a soluble derivative, the peptide or polypeptide can be recovered from the culture, i.e., from the host cell in cases where the NHP peptide or polypeptide is not secreted, and from the culture media in cases where the NHP peptide or polypeptide is secreted by the cells. However, such expression systems also encompass engineered host cells that express a NHP, or functional equivalent, *in situ,* i.e., anchored in the cell membrane. Purification or enrichment of NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e.g.,* in drug screening assays.

The expression systems that may be used for purposes of the invention include but are not limited to microorganisms such as bacteria *(e.g., E. coli, B. subtilis)* transformed with recombinant bacteriophage DNA, plasmid DNA or cosmid DNA expression vectors containing NHP nucleotide sequences; yeast *(e.g., Saccharomyces, Pichia)* transformed with recombinant yeast expression vectors containing NHP nucleotide sequences; insect cell systems infected with recombinant virus expression vectors *(e.g.,* baculovirus) containing NHP sequences; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing NHP nucleotide sequences; or mammalian cell systems *(e.g.,* COS, CHO, BHK, 293, 3T3) harboring recombinant expression constructs containing promoters derived from the genome of mammalian cells *(e.g.,* metallothionein promoter) or from mammalian viruses *(e.g.,* the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E. coli* expression vector pUR278 (Ruther et al., 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, Autographa *californica* nuclear polyhidrosis virus (AcNPV) is used as a vector to express foreign genes. The virus grows in *Spodoptera frugiperda* cells. A NHP coding sequence may be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e.,* virus lacking the proteinaceous coat coded for by the polyhedrin gene). These recombinant viruses are then used to infect Spodoptera frugiperda cells in which the inserted sequence is expressed (*e.g.,* see Smith et al., 1983, J. Virol. 46: 584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, e.g., the late promoter and tripartite leader sequence. This chimeric sequence may then be inserted in the adenovirus genome by in vitro or in vivo recombination. Insertion in a non-essential region of the viral genome (e.g., region E1 or E3) will result in a recombinant virus that is viable and capable of expressing a NHP product in infected hosts (e.g., See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP sequence or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bittner et al., 1987, Methods in Enzymol. 153:516-544).

In addition, a host cell strain may be chosen that modulates the expression of the inserted sequences, or modifies and processes the sequence product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above may be engineered. Rather than using expression vectors which contain viral origins of replication, host cells can be transformed with DNA controlled by appropriate expression control elements (e.g., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, et *al.,* 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147).

Alternatively, any fusion protein may be readily purified by utilizing an antibody specific for the fusion protein being expressed. For example, a system described by Janknecht et *al.* allows for the ready purification of non-denatured fusion proteins expressed in human cell lines (Janknecht, et al., 1991, Proc. Natl. Acad. Sci. USA 88: 8972-8976). In this system, the sequence of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers. NHP products can also be expressed in transgenic animals. Animals of any species, including, but not limited to, worms, mice, rats, rabbits, guinea pigs, pigs, micro-pigs, birds, goats, and non-human primates, e.g., baboons, monkeys, and chimpanzees may be used to generate NHP transgenic animals.

Any technique known in the art may be used to introduce a NHP transgene into animals to produce the founder lines of transgenic animals. Such techniques include, but are not limited to pronuclear microinjection (Hoppe, P.C. and Wagner, T.E., 1989, U.S. Pat. No. 4,873,191); retrovirus mediated gene transfer into germ lines (Van der Putten et al., 1985, Proc. Natl. Acad. Sci., USA 82:6148-6152); gene targeting in embryonic stem cells (Thompson et al., 1989, Cell 56:313-321); electroporation of embryos (Lo, 1983, Mol Cell. Biol. 3:1803-1814); and sperm-mediated gene transfer (Lavitrano et al., 1989, Cell 57:717-723); etc. For a review of such techniques, see Gordon, 1989, Transgenic Animals, Intl. Rev. Cytol. 115:171-229, which is incorporated by reference herein in its entirety.

The present invention provides for transgenic animals that carry the NHP transgene in all their cells, as well as animals which carry the transgene in some, but not all their cells, i.e., mosaic animals or somatic cell transgenic animals. The transgene may be integrated as a single transgene or in concatamers, e.g., head-to-head tandems or head-to-tail tandems. The transgene may also be selectively introduced into and activated in a particular cell type by following, for example, the teaching of Lasko et al., 1992, Proc. Natl. Acad. Sci. USA 89:6232-6236. The regulatory sequences required for such a cell-type specific activation will depend upon the particular cell type of interest, and will be apparent to those of skill in the art.

When it is desired that the NHP transgene be integrated into the chromosomal site of the endogenous NHP gene, gene targeting is preferred. Briefly, when such a technique is to be utilized, vectors containing some nucleotide sequences homologous to the endogenous NHP gene are designed for the purpose of integrating, via homologous recombination with chromosomal sequences, into and disrupting the function of the nucleotide sequence of the endogenous NHP gene (i.e., "knockout" animals).

The transgene may also be selectively introduced into a particular cell type, thus inactivating the endogenous NHP gene in only that cell type, by following, for example, the teaching of Gu et al., 1994, Science, 265:103-106. The regulatory sequences required for such a cell-type specific inactivation will depend upon the particular cell type of interest, and will be apparent to those of skill in the art.

Once transgenic animals have been generated, the expression of the recombinant NHP gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, *in situ* hybridization analysis, and RT-PCR. Samples of NHP gene-expressing tissue, may also be evaluated immunocytochemically using antibodies specific for the NHP transgene product.

### 5.3 ANTIBODIES TO NHPs

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes, as described, below, in Section 5.5, for the evaluation of the effect of test compounds on expression and/or activity of a NHP gene product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, an NHP peptide (e.g., one corresponding to a functional domain of an NHP), truncated NHP polypeptides (NHP in which one or more domains have been deleted), functional equivalents of the NHP or mutants of the NHP. Such host animals may include but are not limited to rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium* parvum. Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures which are herein incorporated by reference in their entirety.

Alternatively, techniques described for the production of single chain antibodies (U.S. Patent 4,946,778; Bird, 1988, Science 242:423-426; Huston et al., 1988, Proc. Natl. Acad. Sci. USA 85:5879-5883; and Ward et al., 1989, Nature 334:544-546) can be adapted to produce single chain antibodies against NHP gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, e.g., Greenspan & Bona, 1993, FASEB J 7(5):437-444; and Nissinoff, 1991, J. Immunol. 147(8):2429-2438). For example antibodies which bind to a NHP domain and competitively inhibit the binding of NHP to its cognate receptor can be used to generate anti-idiotypes that "mimic" the NHP and, therefore, bind and activate or neutralize a receptor. Such anti-idiotypic antibodies or Fab fragments of such anti-idiotypes can be used in therapeutic regimens involving the NHP signaling pathway.

### 5.4 DIAGNOSIS OF ABNORMALITIES RELATED TO A NHP

A variety of methods can be employed for the diagnostic and prognostic evaluation of disorders related to NHP function, and for the identification of subjects having a predisposition to such disorders.

Such methods may, for example, utilize reagents such as the NHP nucleotide sequences described in Section 5.1 and NHP antibodies as described in Section 5.3. Specifically, such reagents may be used, for example, for: (1) the detection of the presence of NHP gene mutations, or the detection of either over- or under-expression of NHP mRNA relative to a given phenotype; (2) the detection of either an over- or an under-abundance of NHP gene product relative to a given phenotype; and (3) the detection of perturbations or abnormalities in any potential signal transduction, metabolic, or catabolic pathway mediated by or involving a NHP.

The methods described herein may be performed, for example, by utilizing pre-packaged diagnostic kits comprising at least one specific NHP nucleotide sequence or NHP antibody reagent described herein, which may be conveniently used, e.g., in clinical settings, to diagnose patients exhibiting body weight disorder abnormalities.

For the detection of NHP mutations, any nucleated cell can be used as a starting source for genomic nucleic acid. For the detection of NHP gene expression or NHP gene products, any cell type or tissue in which the NHP sequence is expressed, such as, for example, brain cells, may be utilized.

Nucleic acid-based detection techniques are described, below, in Section 5.4.1. Peptide detection techniques are described, below, in Section 5.4.2.

### 5.4.1 DETECTION OF NHP SEQUENCES AND TRANSCRIPTS

Mutations within a NHP sequence can be detected by utilizing a number of techniques. Nucleic acid from any nucleated cell can be used as the starting point for such assay techniques, and may be isolated according to standard nucleic acid preparation procedures which are well known to those of skill in the art.

DNA may be used in hybridization or amplification assays of biological samples to detect abnormalities involving NHP gene structure, including point mutations, insertions, deletions and chromosomal rearrangements. Such assays may include, but are not limited to, Southern analyses, single stranded conformational polymorphism analyses (SSCP), and PCR analyses.

Such diagnostic methods for the detection of NHP gene-specific mutations can involve for example, contacting and incubating nucleic acids including recombinant DNA molecules, cloned genes or degenerate variants thereof, obtained from a sample, e.g., derived from a patient sample or other appropriate cellular source, with one or more labeled nucleic acid reagents including recombinant DNA molecules, cloned genes or degenerate variants thereof, as described in Section 5.1, under conditions favorable for the specific annealing of these reagents to their complementary sequences within a given NHP gene. Preferably, the lengths of these nucleic acid reagents are at least about 15 to about 30 nucleotides. After incubation, all non-annealed nucleic acids are removed from the nucleic acid:NHP molecule hybrid. The presence of nucleic acids which have hybridized, if any such molecules exist, is then detected. Using such a detection scheme, the nucleic acid from the cell type or tissue of interest can be immobilized, for example, to a solid support such as a membrane, or a plastic surface such as that on a microtiter plate or polystyrene beads. In this case, after incubation, non-annealed, labeled nucleic acid reagents of the type described in Section 5.1 are easily removed. Detection of the remaining, annealed, labeled NHP nucleic acid reagents is accomplished using standard techniques well-known to those in the art. The NHP sequences to which the nucleic acid reagents have annealed can be compared to the annealing pattern expected from a normal NHP sequence in order to determine whether a NHP gene mutation is present.

Alternative diagnostic methods for the detection of NHP gene specific nucleic acid molecules, in patient samples or other appropriate cell sources, may involve their amplification, e.g., by PCR (the experimental embodiment set forth in Mullis, K.B., 1987, U.S. Patent No. 4,683,202), followed by the detection of the amplified molecules using techniques well known to those of skill in the art. The resulting amplified sequences can be compared to those which would be expected if the nucleic acid being amplified contained only normal copies of a NHP gene in order to determine whether a NHP gene mutation exists.

Additionally, well-known genotyping techniques can be performed to identify individuals carrying NHP gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms which can be utilized for the identification of NHP gene mutations have been described which capitalize on the presence of variable numbers of short, tandemly repeated DNA sequences between the restriction enzyme sites. For example, Weber (U.S. Pat. No. 5,075,217, which is incorporated herein by reference in its entirety) describes a DNA marker based on length polymorphisms in blocks of (dC-dA)n-(dG-dT)n short tandem repeats. The average separation of (dC-dA)n-(dG-dT)n blocks is estimated to be 30,000-60,000 bp. Markers which are so closely spaced exhibit a high frequency co-inheritance, and are extremely useful in the identification of genetic mutations, such as, for example, mutations within a given NHP gene, and the diagnosis of diseases and disorders related to NHP mutations.

Also, Caskey et al. (U.S. Pat. No. 5,364,759, which is incorporated herein by reference in its entirety) describe a DNA profiling assay for detecting short tri and tetra nucleotide repeat sequences. The process includes extracting the DNA of interest, such as the NHP gene, amplifying the extracted DNA, and labeling the repeat sequences to form a genotypic map of the individual's DNA.

The level of NHP gene expression can also be assayed by detecting and measuring NHP transcription. For example, RNA from a cell type or tissue known, or suspected to express the NHP sequence, such as brain, may be isolated and tested utilizing hybridization or PCR techniques such as are described, above. The isolated cells can be derived from cell culture or from a patient. The analysis of cells taken from culture may be a necessary step in the assessment of cells to be used as part of a cell-based gene therapy technique or, alternatively, to test the effect of compounds on the expression of the NHP sequence. Such analyses may reveal both quantitative and qualitative aspects of the expression pattern of the NHP gene, including activation or inactivation of NHP gene expression.

In one embodiment of such a detection scheme, cDNAs are synthesized from the RNAs of interest (e.g., by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (e.g., primers) in the reverse transcription and nucleic acid amplification steps of this method are chosen from among the NHP nucleic acid reagents described in Section 5.1. The preferred lengths of such nucleic acid reagents are at least about 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining, by utilizing any other suitable nucleic acid staining method, or by sequencing.

Additionally, it is possible to perform such NHP gene expression assays "in situ", i.e., directly upon tissue sections (fixed and/or frozen) of patient tissue obtained from biopsies or resections, such that no nucleic acid purification is necessary. Nucleic acid reagents such as those described in Section 5.1 may be used as probes and/or primers for such in situ procedures (See, for example, Nuovo, G.J., 1992, "PCR In Situ Hybridization: Protocols And Applications", Raven Press, NY).

Alternatively, if a sufficient quantity of the appropriate cells can be obtained, standard Northern analysis can be performed to determine the level and tissue distribution of mRNA expression of a NHP gene.

### 5.4.2 DETECTION OF NHP GENE PRODUCTS

Antibodies directed against wild type or mutant NHP gene products or conserved variants or peptide fragments thereof, which are discussed, above, in Section 5.3, may also be used as diagnostics and prognostics, as described herein. Such diagnostic methods, may be used to detect abnormalities in the level of NHP gene expression, or abnormalities in the structure and/or temporal, tissue, cellular, or subcellular location of the NHP, and may be performed *in vivo* or *in vitro,* such as, for example, on biopsy tissue.

For example, antibodies directed to epitopes of an NHP can be used *in vivo* to detect the pattern and level of expression of the NHP in the body. Such antibodies can be labeled, e.g., with a radio-opaque or other appropriate compound and injected into a subject in order to visualize binding to the NHP expressed in the body using methods such as X-rays, CAT-scans, or MRI. Labeled antibody fragments, e.g., the Fab or single chain antibody comprising the smallest portion of the antigen binding region, are preferred for this purpose to promote crossing the blood-brain barrier and permit labeling of NHPs expressed in the brain.

Additionally, any NHP fusion protein or NHP conjugated protein whose presence can be detected, can be administered. For example, NHP fusion or conjugated proteins labeled with a radio-opaque or other appropriate compound can be administered and visualized *in vivo,* as discussed, above for labeled antibodies. Further such NHP fusion proteins (such as alkaline phosphatase- NHP or NHP-alkaline phosphatase) can be utilized for *in vitro* diagnostic procedures.

Alternatively, immunoassays or fusion protein detection assays, as described above, can be utilized on biopsy and autopsy samples *in vitro* to permit assessment of the expression pattern of the NHP. Such assays are not confined to the use of antibodies that define a NHP domain, but can include the use of antibodies directed to epitopes of any domain of a NHP. The use of each or all of these labeled antibodies will yield useful information regarding translation and intracellular transport of the NHP to the cell surface and can identify defects in processing.

The tissue or cell type to be analyzed will generally include those which are known, or suspected, to express the NHP sequence, such as, for example, epithelial cells, kidney cells, placenta cells, brain cells, etc. The protein isolation methods employed herein may, for example, be such as those described in Harlow and Lane (Harlow, E. and Lane, D., 1988, "Antibodies: A Laboratory Manual", Cold Spring Harbor Laboratory Press, Cold Spring Harbor, New York), which is incorporated herein by reference in its entirety. The isolated cells can be derived from cell culture or from a patient. The analysis of cells taken from culture may be a necessary step in the assessment of cells that could be used as part of a cell-based gene therapy technique or, alternatively, to test the effect of compounds on the expression of a NHP sequence.

For example, antibodies, or fragments of antibodies, such as those described above in Section 5.3 are useful in the present invention, and may be used to quantitatively or qualitatively detect the presence of NHP products, or conserved variants or peptide fragments thereof. This can be accomplished, for example, by immunofluorescence techniques employing a fluorescently labeled antibody (see below, this Section) coupled with light microscopic, flow cytometric, or fluorimetric detection. Such techniques are especially preferred if such NHP products are at least transiently present on the cell surface.

The antibodies (or fragments thereof) or NHP fusion or conjugated proteins useful in the present invention may, additionally, be employed histologically, as in immunofluorescence, immunoelectron microscopy or non-immuno assays, for in situ detection of NHP gene products or conserved variants or peptide fragments thereof, or to assay NHP binding (in the case of labeled NHP-fusion protein).

In situ detection may be accomplished by removing a histological specimen from a patient, and applying thereto a labeled antibody or fusion protein of the present invention. The antibody (or fragment) or fusion protein is preferably applied by overlaying the labeled antibody (or fragment) onto a biological sample. Through the use of such a procedure, it is possible to determine not only the presence of the NHP product, or conserved variants or peptide fragments, or NHP binding, but also its distribution in the examined tissue. Using the present invention, those of ordinary skill will readily perceive that any of a wide variety of histological methods (such as staining procedures) can be modified in order to achieve such *in situ* detection.

Immunoassays and non-immunoassays for NHP products, or conserved variants or peptide fragments thereof, will typically comprise incubating a sample, such as a biological fluid, a tissue extract, freshly harvested cells, or lysates of cells which have been incubated in cell culture, in the presence of a detectably labeled antibody capable of identifying NHP products or conserved variants or peptide fragments thereof, and detecting the bound antibody by any of a number of techniques well-known in the art. Alternatively, the labeled antibody can be directed against an antigenic tag that has been directly or indirectly attached to a NHP.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled NHP antibody or NHP receptor fusion protein. The solid phase support may then be washed with the buffer a second time to remove unbound antibody or fusion protein. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of NHP antibody or NHP ligand fusion protein may be determined according to well known methods. Those skilled in the art will be able to determine operative and optimal assay conditions for each determination by employing routine experimentation.

With respect to antibodies, one of the ways in which the NHP antibody can be detectably labeled is by linking the same to an enzyme and use in an enzyme immunoassay (EIA) (Voller, A., "The Enzyme Linked Immunosorbent Assay (ELISA)", 1978, Diagnostic Horizons 2:1-7, Microbiological Associates Quarterly Publication, Walkersville, MD); Voller, A. et al., 1978, J. Clin. Pathol. 31:507-520; Butler, J.E., 1981, Meth. Enzymol. 73:482-523; Maggio, E. (ed.), 1980, Enzyme Immunoassay, CRC Press, Boca Raton, FL,; Ishikawa, E. et al., (eds.), 1981, Enzyme Immunoassay, Kgaku Shoin, Tokyo). The enzyme that is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect NHP through the use of a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

### 5.5 SCREENING ASSAYS FOR COMPOUNDS THAT MODULATE NHP EXPRESSION OR ACTIVITY

The following assays are designed to identify compounds that interact with (e.g., bind to) NHPs, compounds that interfere with the interaction of an NHP with its receptor, and to compounds that modulate the activity of NHP gene expression (i.e., modulate the level of NHP gene expression) or modulate the levels of NHP in the body. Assays may additionally be utilized which identify compounds that bind to NHP gene regulatory sequences (e.g., promoter sequences) and, consequently, may modulate NHP gene expression. See e.g., Platt, K.A., 1994, J. Biol. Chem. 269:28558-28562, which is incorporated herein by reference in its entirety.

The compounds which may be screened in accordance with the invention include but are not limited to peptides, antibodies and fragments thereof, and other organic compounds (e.g., peptidomimetics) that bind to a NHP and either mimic the activity triggered by the natural product (i.e., agonists) or inhibit the activity triggered by the natural ligand (i.e., antagonists); as well as peptides, antibodies or fragments thereof, and other organic compounds that mimic the NHP (or a portion thereof) and bind to and "activate" or "neutralize" the natural receptor.

Such compounds may include, but are not limited to, peptides such as, for example, soluble peptides, including but not limited to members of random peptide libraries; (see, *e.g.,* Lam, K.S. et al., 1991, Nature 354:82-84; Houghten, R. et al., 1991, Nature 354:84-86), and combinatorial chemistry-derived molecular library made of D- and/or L- configuration amino acids, phosphopeptides (including, but not limited to members of random or partially degenerate, directed phosphopeptide libraries; see, e.g., Songyang, Z. et al., 1993, Cell 72:767-778), antibodies (including, but not limited to, polyclonal, monoclonal, humanized, anti-idiotypic, chimeric or single chain antibodies, and FAb, F(ab')₂ and FAb expression library fragments, and epitope-binding fragments thereof), and small organic or inorganic molecules.

Other compounds which can be screened in accordance with the invention include but are not limited to small organic molecules that are able to cross the blood-brain barrier, gain entry into an appropriate cell (e.g., in the choroid plexus, pituitary, the hypothalamus, etc.) and affect the expression of a NHP gene or some other gene involved in a NHP mediated pathway (e.g., by interacting with the regulatory region or transcription factors involved in gene expression); or such compounds that affect or substitute for the activity of the NHP or the activity of some other intracellular factor involved in a NHP signal transduction, catabolic, or metabolic pathways.

Computer modeling and searching technologies permit identification of compounds, or the improvement of already identified compounds, that can modulate NHP expression or activity. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site (or binding site), either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential NHP modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active sites (or binding sites) of a NHP, and related transduction and transcription factors will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen, et al., 1988, Acta Pharmaceutical Fennica 97:159-166; Ripka, New Scientist 54-57 (June 16, 1988); McKinaly and Rossmann, 1989, Annu. Rev. Pharmacol. Toxiciol. 29:111-122; Perry and Davies, OSAR: Quantitative Structure-Activity Relationships in Drug Design pp. 189-193 (Alan R. Liss, Inc. 1989); Lewis and Dean, 1989 Proc. R. Soc. Lond. 236:125-140 and 141-162; and, with respect to a model receptor for nucleic acid components, Askew, et al., 1989, J. Am. Chem. Soc. 111:1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

Although described above with reference to design and generation of compounds which could alter binding, one could also screen libraries of known compounds, including natural products or synthetic chemicals, and biologically active materials, including proteins, for compounds which are inhibitors or activators.

Cell-based systems can also be used to identify compounds that bind (or mimic) NHPs as well as assess the altered activity associated with such binding in living cells. One tool of particular interest for such assays is green fluorescent protein which is described, *inter alia,* in U.S. Patent No. 5,625,048, herein incorporated by reference. Cells that may be used in such cellular assays include, but are not limited to, leukocytes, or cell lines derived from leukocytes, lymphocytes, stem cells, including embryonic stem cells, and the like. In addition, expression host cells *(e.g.,* B95 cells, COS cells, CHO cells, OMK cells, fibroblasts, Sf9 cells) genetically engineered to express a functional NHP of interest and to respond to activation by the test, or natural, ligand, as measured by a chemical or phenotypic change, or induction of another host cell gene, can be used as an end point in the assay.

Compounds identified via assays such as those described herein may be useful, for example, in elucidating the biological function of a NHP gene product. Such compounds can be administered to a patient at therapeutically effective doses to treat any of a variety of physiological or mental disorders. A therapeutically effective dose refers to that amount of the compound sufficient to result in any amelioration, impediment, prevention, or alteration of any biological symptom.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients. Thus, the compounds and their physiologically acceptable salts and solvates may be formulated for administration by inhalation or insufflation (either through the mouth or the nose) or oral, buccal, parenteral, intracranial, intrathecal, or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multidose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 5.5.1 IN VITRO SCREENING ASSAYS FOR COMPOUNDS THAT BIND TO NHPs

*In vitro* systems may be designed to identify compounds capable of interacting with (e.g., binding to) or mimicking NHPs. The compounds identified can be useful, for example, in modulating the activity of wild type and/or mutant NHP gene products; can be useful in elaborating the biological function of the NHP; can be utilized in screens for identifying compounds that disrupt normal NHP interactions; or may themselves disrupt or activate such interactions.

The principle of the assays used to identify compounds that bind to NHPs, or NHP receptors, involves preparing a reaction mixture of an NHP and the test compound under conditions and for a time sufficient to allow the two components to interact and bind, thus forming a complex which can be removed and/or detected in the reaction mixture. The NHP species used can vary depending upon the goal of the screening assay. For example, where agonists of the natural receptor are desired, the full length NHP, or a soluble truncated NHP, a peptide, or fusion protein containing one or more NHP domains fused to a protein or polypeptide that affords advantages in the assay system (e.g., labeling, isolation of the resulting complex, etc.) can be utilized. Where compounds that directly interact with the NHP are sought, peptides corresponding to the NHP and fusion proteins containing NHPs can be used.

The screening assays can be conducted in a variety of ways. For example, one method to conduct such an assay would involve anchoring the NHP, polypeptide, peptide, or fusion protein therefrom, or the test substance onto a solid phase and detecting NHP/test compound complexes anchored on the solid phase at the end of the reaction. In one embodiment of such a method, the NHP reactant may be anchored onto a solid surface, and the test compound, which is not anchored, may be labeled, either directly or indirectly.

In practice, microtiter plates may conveniently be utilized as the solid phase. The anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the nonimmobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously nonimmobilized component is pre-labeled, the detection of label immobilized on the surface indicates that complexes were formed. Where the previously nonimmobilized component is not pre-labeled, an indirect label can be used to detect complexes anchored on the surface; e.g., using a labeled antibody specific for the previously nonimmobilized component (the antibody, in turn, may be directly labeled or indirectly labeled with a labeled anti-Ig antibody).

Alternatively, a reaction can be conducted in a liquid phase, the reaction products separated from unreacted components, and complexes detected; e.g., using an immobilized antibody specific for a NHP protein, polypeptide, peptide or fusion protein or the test compound to anchor any complexes formed in solution, and a labeled antibody specific for the other component of the possible complex to detect anchored complexes.

Alternatively, cell-based assays can be used to identify compounds that interact with NHP. To this end, cell lines that express NHP or NHP receptor, or cell lines *(e.g.,* COS cells, CHO cells, fibroblasts, etc.) that have been genetically engineered to express NHP or NHP receptor (*e.g.,* by transfection or transduction of NHP DNA) can be used. Interaction of the test compound with, for example, a NHP receptor expressed by the host cell can be determined by comparison or competition with native NHP.

### 5.5.2 ASSAYS FOR INTRACELLULAR PROTEINS THAT ARE ACTIVATED BY NHP BINDING

Any method suitable for detecting protein-protein interactions may be employed for identifying transmembrane proteins or intracellular proteins that interact with a NHP receptor. Among the traditional methods which may be employed are co-immunoprecipitation, crosslinking and co-purification through gradients or chromatographic columns of cell lysates or proteins obtained from cell lysates and a NHP receptor to identify proteins in the lysate that interact with the NHP receptor. For these assays, the NHP component can be a full length NHP receptor, a soluble derivative lacking the membrane-anchoring region (e.g., a truncated NHP receptor in which a TM is deleted resulting in a truncated molecule containing a ECD fused to a CD), a peptide corresponding to a CD or a fusion protein containing a CD of a NHP receptor. Once isolated, such an intracellular protein can be identified and can, in turn, be used, in conjunction with standard techniques, to identify proteins with which it interacts. For example, at least a portion of the amino acid sequence of an intracellular protein which interacts with a NHP receptor can be ascertained using techniques well known to those of skill in the art, such as via the Edman degradation technique. (See, e.g., Creighton, 1983, "Proteins: Structures and Molecular Principles", W.H. Freeman & Co., N.Y., pp.34-49). The amino acid sequence obtained may be used as a guide for the generation of oligonucleotide mixtures that can be used to screen for gene sequences encoding such intracellular proteins. Screening may be accomplished, for example, by standard hybridization or PCR techniques. Techniques for the generation of oligonucleotide mixtures and the screening are well-known. (See, e.g., Ausubel, *supra*, and PCR Protocols: A Guide to Methods and Applications, 1990, Innis, M. et al., eds. Academic Press, Inc., New York).

Additionally, methods may be employed which result in the simultaneous identification of genes that encode the transmembrane or intracellular proteins interacting with the NHP receptor. These methods include, for example, probing expression, libraries, in a manner similar to the well known technique of antibody probing of λgt11 libraries, using labeled NHP protein, or an NHP polypeptide, peptide or fusion protein, e.g., an NHP polypeptide or NHP domain fused to a marker (e.g., an enzyme, fluor, luminescent protein, or dye), or an Ig-Fc domain.

One method which detects protein interactions *in vivo,* the two-hybrid system, is described in detail for illustration only and not by way of limitation. One version of this system has been described (Chien et al., 1991, Proc. Natl. Acad. Sci. USA, 88:9578-9582) and is commercially available from Clontech (Palo Alto, CA).

Briefly, utilizing such a system, plasmids are constructed that encode two hybrid proteins: one plasmid consists of nucleotides encoding the DNA-binding domain of a transcription activator protein fused to a nucleotide sequence encoding an NHP receptor, an NHP, or polypeptide, peptide, or fusion protein therefrom, and the other plasmid consists of nucleotides encoding the transcription activator protein's activation domain fused to a cDNA encoding an unknown protein which has been recombined into this plasmid as part of a cDNA library. The DNA-binding domain fusion plasmid and the cDNA library are transformed into a strain of the yeast *Saccharomyces cerevisiae* that contains a reporter gene *(e.g.,* HBS or *lacZ*) whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, a NHP or NHP receptor can be used as the bait gene product. Total genomic or cDNA sequences are fused to the DNA encoding an activation domain. This library and a plasmid encoding a hybrid of a bait NHP gene product fused to the DNA-binding domain are cotransformed into a yeast reporter strain, and the resulting transformants are screened for those that express the reporter gene. For example, and not by way of limitation, a bait NHP gene sequence, such as the open reading frame of a NHP (or a domain of a NHP) can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait NHP gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait NHP gene-GAL4 fusion plasmid into a yeast strain which contains a lacZ gene driven by a promoter which contains GAL4 activation sequence. A cDNA encoded protein, fused to GAL4 transcriptional activation domain, that interacts with bait NHP gene product will reconstitute an active GAL4 protein and thereby drive expression of the HIS3 gene. Colonies which express HIS3 can be detected by their growth on petri dishes containing semi-solid agar based media lacking histidine. The cDNA can then be purified from these strains, and used to produce and isolate the bait NHP gene-interacting protein using techniques routinely practiced in the art.

### 5.5.3 ASSAYS FOR COMPOUNDS THAT INTERFERE WITH NHP RECEPTOR/INTRACELLULAR OR NHP/TRANSMEMBRANE MACROMOLECULE INTERACTION

Macromolecules that interact with NHPs or NHP receptors are referred to, for purposes of this discussion, as "binding partners". These binding partners are likely to be involved in the NHP mediated biological pathways. Therefore, it is desirable to identify compounds that interfere with or disrupt the interaction of such binding partners which may be useful in regulating or augmenting NHP activity in the body and/or controlling disorders associated with NHP activity (or a deficiency thereof).

The basic principle of the assay systems used to identify compounds that interfere with the interaction between a NHP or NHP receptor (collectively, the NHP moiety), and its binding partner or partners involves preparing a reaction mixture containing NHP or NHP receptor, or NHP polypeptides, peptides or fusion proteins as described in Sections 5.5.1 and 5.5.2 above, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the NHP moiety and its binding partner. Control reaction mixtures are incubated without the test compound or with a placebo. The formation of any complexes between the NHP moiety and the binding partner is then detected. The formation of a complex in the control reaction, but not in the reaction mixture containing the test compound, indicates that the compound interferes with the interaction of the NHP moiety and the interactive binding partner. Additionally, complex formation within reaction mixtures containing the test compound and normal NHP protein may also be compared to complex formation within reaction mixtures containing the test compound and a mutant NHP. This comparison may be important in those cases wherein it is desirable to identify compounds that specifically disrupt interactions of mutant, or mutated, NHPs but not normal NHPs.

The assay for compounds that interfere with the interaction of the NHP and binding partners can be conducted in a heterogeneous or homogeneous format. Heterogeneous assays involve anchoring either the NHP moiety or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction by competition can be identified by conducting the reaction in the presence of the test substance; i.e., by adding the test substance to the reaction mixture prior to, or simultaneously with, a NHP moiety and interactive binding partner. Alternatively, test compounds that disrupt preformed complexes, e.g. compounds with higher binding constants that displace one of the components from the complex, can be tested by adding the test compound to the reaction mixture after complexes have been formed. The various formats are described briefly below.

In a heterogeneous assay system, either a NHP moiety or an interactive binding partner, is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of the NHP moiety or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (e.g., by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, may be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds which inhibit complex or which disrupt preformed complexes can be identified.

In an alternate embodiment of the invention, a homogeneous assay can be used. In this approach, a preformed complex of a NHP moiety and an interactive binding partner is prepared in which either the NHP moiety or its binding partners is labeled, but the signal generated by the label is quenched due to formation of the complex (see, e.g., U.S. Patent No. 4,190,496 by Rubenstein which utilizes this approach for immunoassays). The addition of a test substance that competes with and displaces one of the species from the preformed complex will result in the generation of a signal above background. In this way, test substances which disrupt NHP/intracellular binding partner interaction can be identified.

A NHP fusion can be prepared for immobilization. For example, a NHP or a peptide fragment can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art and described above, in Section 5.3. This antibody can be labeled with the radioactive isotope ¹²⁵I, for example, by methods routinely practiced in the art. In a heterogeneous assay, e.g., the GST-NHP fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between a NHP moiety and the interactive binding partner can be detected by measuring the amount of radioactivity that remains associated with the glutathione-agarose beads. A successful inhibition of the interaction by the test compound will result in a decrease in measured radioactivity.

Alternatively, the GST-NHP moiety fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the NHP moiety/binding partner interaction can be detected by adding the labeled antibody and measuring the radioactivity associated with the beads.

These same techniques can be employed using peptide fragments that correspond to the binding domains of a NHP moiety and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensatory mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a relatively short, labeled peptide comprising the binding domain may remain associated with the solid material, which can be isolated and identified by amino acid sequencing. Also, once the gene coding for the intracellular binding partner is obtained, short gene segments can be engineered to express peptide fragments of the protein, which can then be tested for binding activity and purified or synthesized.

For example, and not by way of limitation, a NHP moiety can be anchored to a solid material as described, above, by making a GST-NHP moiety fusion protein and allowing it to bind to glutathione agarose beads. The interactive binding partner can be labeled with a radioactive isotope, such as ³⁵S, and cleaved with a proteolytic enzyme such as trypsin. Cleavage products can then be added to the anchored GST-NHP moiety fusion protein and allowed to bind. After washing away unbound peptides, labeled bound material, representing the intracellular binding partner binding domain, can be eluted, purified, and analyzed for amino acid sequence by well-known methods. Peptides so identified can be produced synthetically or fused to appropriate facilitative proteins using recombinant DNA technology.

The present invention is not to be limited in scope by the specific embodiments described herein, which are intended as single illustrations of individual aspects of the invention. Indeed, various modifications of the invention, in addition to those shown and described herein will become apparent to those skilled in the art from the foregoing description and accompanying drawings.

## Claims

1. An isolated nucleic acid molecule comprising a nucleotide sequence disclosed in SEQ ID NO: 3.

2. An isolated nucleic acid molecule comprising a nucleotide sequence that encodes the amino acid sequence shown in SEQ ID NO: 4.

3. A polypeptide encoded by a nucleic acid as claimed in claim 1 or claim 2.

4. An antibody that specifically recognises one or more epitopes of a polypeptide as claimed in claim 3, or an epitope-binding fragment or a derivative of said antibody that specifically recognises one or more epitopes of a polypeptide as claimed in claim 3, for example, a polyclonal or monoclonal antibody, a single chain antibody, a Fab fragment, a F(ab')2, a fragment produced by a Fab expression library, or an anti-idiotypic (anti-Id) antibody.

5. A host cell that comprises a nucleic acid as claimed in claim 1 or claim 2 and is capable of expressing the polypeptide encoded by said nucleic acid, optionally on the surface of said host cell.

6. A non human transgenic animal that expresses a nucleic acid as claimed in any one of claims 1 to 2.

## Patentansprüche

1. Ein isoliertes Nukleinsäuremolekül umfassend eine Nukleotidsequenz offenbart in SEQ ID NO: 3.

2. Ein isoliertes Nukleinsäuremolekül umfassend eine Nukleotidsequenz, die für die in SEQ ID NO: 4 gezeigte Aminosäuresequenz codiert.

3. Ein Polypeptid, das durch eine Nukleinsäure codiert wird wie in Anspruch 1 oder Anspruch 2 beansprucht.

4. Ein Antikörper, der spezifisch ein oder mehrere Epitope eines Polypeptids wie in Anspruch 3 erkennt, oder ein Epitop bindendes Fragment oder ein Derivat des Antikörpers, der spezifisch ein oder mehrere Epitope eines Polypeptids wie in Anspruch 3 beansprucht erkennt, beispielsweise ein polyklonaler oder monoklonaler Antikörper, ein einsträngiger Antikörper, ein Fab-Fragment, ein F(ab')2, ein Fragment hergestellt durch eine Fab-Expressionsbibliothek, oder ein antiidiotypischer (anti-Id) Antikörper.

5. Ein Wirtszelle, die eine Nukleinsäure umfasst wie in Anspruch 1 oder Anspruch 2 beansprucht und in der Lage ist, das Polypeptid zu exprimieren, das durch die Nukleinsäure codiert wird, gegebenenfalls auf der Oberfläche der Wirtszelle.

6. Ein nicht menschliches transgenes Tier, das eine Nukleinsäure wie in einem der Ansprüche 1 und 2 beansprucht exprimiert.

## Revendications

1. Molécule d'acide nucléique isolée comprenant une séquence de nucléotides décrite dans SEQ ID N° 3.

2. Molécule d'acide nucléique isolée comprenant une séquence de nucléotides qui code pour la séquence d'acides aminés représentée dans SEQ ID N° 4.

3. Polypeptide codé par un acide nucléique selon la revendication 1 ou 2.

4. Anticorps qui reconnaît spécifiquement un ou plusieurs épitopes d'un polypeptide selon la revendication 3, ou un fragment de liaison d'épitope ou un dérivé dudit anticorps qui reconnaît spécifiquement un ou plusieurs épitopes d'un polypeptide selon la revendication 3, par exemple, un anticorps polyclonal ou monoclonal, un anticorps à chaîne unique, un fragment Fab, un F(ab')2, un fragment produit par une banque d'expression Fab ou un anticorps anti-idiotypique (anti-Id).

5. Cellule hôte qui comprend un acide nucléique selon la revendication 1 ou 2 et qui est capable d'exprimer le polypeptide codé par ledit acide nucléique, éventuellement sur la surface de ladite cellule hôte.

6. Animal transgénique non humain qui exprime un acide nucléique selon l'une quelconque des revendications 1 ou 2.
